(19) Europäisches Patentamt / European Patent Office / Office européen des brevets

(11) EP 3 035 244 A1

(12) EUROPEAN PATENT APPLICATION

(43) Date of publication:
22.06.2016 Bulletin 2016/25

(51) Int Cl.:
G06K 9/00 (2006.01)   H01L 51/52 (2006.01)
H01L 27/32 (2006.01)

(21) Application number: 15200086.5

(22) Date of filing: 15.12.2015

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 17.12.2014 JP 2014254828

(71) Applicant: Seiko Epson Corporation
Tokyo 163 (JP)

(72) Inventor: Tsuchiya, Hitoshi
Nagano, 392-8502 (JP)

(74) Representative: Hoffmann Eitle
Patent- und Rechtsanwälte PartmbB
Arabellastraße 30
81925 München (DE)

(54) IMAGE ACQUISITION APPARATUS, BIOLOGICAL BODY INFORMATION ACQUISITION APPARATUS, AND ELECTRONIC APPARATUS

(57) An image acquisition apparatus includes an imaging section including a light receiving device and a light emitting section that is superimposed on the imaging section and illuminates a subject. The light emitting section (110) includes a light transmissive first substrate (111), a light emitting device provided on the device substrate, a barrier section (23) as a resin layer that defines a light emitting region in the light emitting device, and a light transmissive portion (112) that transmits light reflected off the illuminated subject and guides the reflected light to the light receiving device, and the barrier section (23) contains an insulating resin material and a light absorbing material. The barrier section (23) absorbs light that is emitted from the light emitting region and could undesirably leak toward the light transmissive portion.

FIG. 7B

EP 3 035 244 A1

**Description**

BACKGROUND

1. Technical Field

The present invention relates to an image acquisition apparatus, a biological body information acquisition apparatus, and an electronic apparatus.

2. Related Art

An imaging apparatus that images a subject to acquire an image has been disclosed (JP-A-2014-67577). The imaging apparatus exemplified in JP-A-2014-67577 has a structure in which a light receiving section, a light blocking section, a light emitting section, and a light collecting section are stacked in this order on each other. After a subject is illuminated with imaging light outputted from the light emitting section and light from the subject incident on the light collecting section is collected by the light collecting section, the collected light passes through openings provided in each of the light emitting section and the light blocking section and reaches the light receiving section, which is located in the lowest layer. The light receiving section has a plurality of light receiving devices and performs image processing on the intensity of the light originating from the subject and incident on the plurality of light receiving devices to produce image information on the subject.

The light emitting section exemplified in the imaging apparatus described above includes first electrode layers, a second electrode layer, and a light emitting layer sandwiched between the two electrode layers and made of an organic EL (electroluminescence) material. A light emitting region in the light emitting section is defined by an insulating layer so provided that it surrounds a region where each of the first electrode layers is in contact with the light emitting layer. JP-A-2014-67577 shows an example in which the positions of the light emitting regions relative to the optical axes of lenses as the light collecting section are so defined that only the light incident from the illuminated subject is incident on light receiving surfaces of the light receiving devices but the imaging light emitted from the light emitting section and reflected off the surfaces of the lenses is not incident on the light receiving surfaces.

The second electrode layer of the light emitting section in the imaging apparatus described in JP-A-2014-67577, however, is provided as a common electrode common to the plurality of first electrode layers and has portions that are provided in the regions other than the light emitting regions and face the first electrode layers via the insulating layers. The first electrode layers have surfaces having light reflectivity and are made of a material having a refractive index different from those of the insulating layers and the second electrode layer. The light emitted from the light emitting layer could undesirably be reflected off the surfaces of the first electrode layers other than the light emitting regions and further reflected again off the interfaces between the insulating layers and the second electrode layer, possibly resulting in what is called stray light. The thus formed stray light, when it is incident on the light receiving surface of the light receiving device, affects the intensity of the light incident from the subject, possibly resulting in an unclear image of the subject. The stray light is formed not only of the light reflected off the interfaces between the insulating layers and the second electrode layer but also of light refracted at or reflected off the interface of members which are present between the light collecting section and the light receiving section and through which light passes.

SUMMARY

An advantage of some aspects of the invention is to solve at least a part of the problems described above, and the invention can be implemented as the following forms or application examples.

Application Example

An image acquisition apparatus according to this application example includes an imaging section including a light receiving device and a light emitting section that is superimposed on the imaging section and illuminates a subject. The light emitting section includes a light transmissive first substrate, a light emitting device provided on the first substrate, a resin layer that defines a light emitting region in the light emitting device, and a light transmissive portion that transmits light reflected off the illuminated subject and guides the reflected light to the light receiving device, and the resin layer contains an insulating resin material and a light absorbing material.

According to this application example, since the resin layer that defines the light emitting region of the light emitting device contains a light absorbing material, light that is emitted from the light emitting region and could undesirably leak to the light transmissive portion is absorbed by the light absorbing material in the resin layer. Stray light incident through the light transmissive portion on the light receiving device in the imaging section other than light reflected off an

EP 3 035 244 A1

illuminated subject can be reduced, whereby an image acquisition apparatus capable of acquiring a clear image can be provided.

In the image acquisition apparatus according to the application example described above, the light absorbing material may be carbon black or a Ti-based black pigment.

According to the configuration described above, light that is emitted from the light emitting region of the light emitting device and could undesirably leak to the light transmissive portion can be adequately absorbed by the light absorbing material.

In the image acquisition apparatus according to the application example described above, it is preferable that the light emitting device has a first electrode and a second electrode so disposed on the first substrate that the electrodes face each other and a light emission function layer disposed between the first electrode and the second electrode, and that the resin layer is provided between the first electrode and the second electrode and so disposed that the resin layer defines a region where the first electrode is in contact with the light emission function layer and encloses an outer edge of the first electrode in a plan view.

The configuration described above does not allow the light emitted from the light emitting region of the light emitting device to be reflected off the interface between the resin layer and the first electrode or the interface between the resin layer and the second electrode and therefore does not allow the light to leak toward the light transmissive portion. That is, light that is emitted from the light emitting region of the light emitting device and could undesirably leak to the light transmissive portion can be reliably absorbed by the resin layer.

In the image acquisition apparatus according to the application example described above, it is preferable that the light emitting device has a reflection layer disposed between the first substrate and the first electrode, and that the first electrode has light transmissivity and is layered on the reflection layer.

According to the configuration described above, illumination light emitted from the light emitting device can be efficiently extracted through the second electrode. That is, the light emitting section can efficiently illuminate a subject.

In the image acquisition apparatus according to the application example described above, the light emitting device may have a reflection layer disposed between the first substrate and the first electrode, and the first electrode may have light transmissivity and may be layered on the reflection layer via an interlayer insulating film.

According to the configuration described above, adjustment of the film thickness of the interlayer insulating film between the reflection layer and the first electrode allows extraction of the illumination light with enhanced optical intensity at a specific wavelength through the second electrode.

It is preferable that the image acquisition apparatus according to the application example described above further includes a light collecting section superimposed on the light emitting section, and the light collecting section preferably includes a light transmissive second substrate and a collector lens provided on the second substrate on a surface thereof facing the light emitting section and in a position where the collector lens faces the light transmissive portion.

According to the configuration described above, when a subject is brought onto the side where the light collecting section is present relative to the light emitting section, light reflected off an illuminated subject can be collected with the collector lens and guided to the light receiving device. That is, an image acquisition apparatus capable of acquiring a bright, clear image can be provided.

It is preferable that the image acquisition apparatus according to the application example described above further includes a light blocking section disposed between the light emitting section and the imaging section, and the light blocking section preferably includes a light transmissive third substrate, a light blocking layer provided on the third substrate on a surface thereof facing the imaging section, and an opening formed in the light blocking layer and in a position where the opening faces the light receiving device.

According to the configuration described above, the light blocking layer of the light blocking section can block stray light produced in the course of passage of light reflected off an illuminated subject from the light emitting section to the imaging section and when the light passes through the interface between members having different refractive indices and is reflected off or refracted by the interface. That is, influence of the stray light on the light reflected off the illuminated subject can be further reduced, whereby an image acquisition apparatus capable of acquiring a clearer image can be provided.

The image acquisition apparatus according to the application example described above may further include a light collecting section and a light blocking section disposed between the light emitting section and the imaging section. The light collecting section may include a light transmissive second substrate and a collector lens provided on the second substrate on a surface thereof opposite the light emitting section and in a position where the collector lens faces the light transmissive portion, and the light blocking section may include a light transmissive third substrate, a light blocking layer provided on the third substrate on a surface thereof facing the imaging section, and an opening formed in the light blocking layer and in a position where the opening faces the light receiving device.

According to the configuration described above, providing the light collecting section and the light blocking section between the light emitting section and the imaging section allows an image acquisition apparatus capable of

3

acquiring a bright, clearer image to be provided.

In the image acquisition apparatus according to the application example described above, it is preferable that a center of a light receiving surface of the light receiving device, a center of the opening in the light blocking layer, and a center of the light transmissive portion in the light emitting section roughly coincide with one another in a plan view.

According to the configuration described above, light reflected off an illuminated subject can be efficiently guided to the light receiving device in the imaging section.

Application Example

A biological body information acquisition apparatus according to this application example includes an imaging section including a light receiving device and a light emitting section that is superimposed on the imaging section and illuminates a biological body. The light emitting section includes a light transmissive first substrate, a light emitting device that is provided on the first substrate and emits near infrared light, a resin layer that defines a light emitting region in the light emitting device, and a light transmissive portion that transmits light reflected off the illuminated biological body and guides the reflected light to the light receiving device, and the resin layer contains an insulating resin material and a light absorbing material.

According to this application example, since the resin layer that defines the light emitting region of the light emitting device contains a light absorbing material, light that is emitted from the light emitting region and could undesirably leak to the light transmissive portion is absorbed by the light absorbing material in the resin layer. Stray light incident through the light transmissive portion on the light receiving device in the imaging section other than light reflected off an illuminated subject can be reduced, whereby a biological body information acquisition apparatus capable of acquiring clear biological body information can be provided.

In the biological body information acquisition apparatus according to the application example described above, it is preferable that the light absorbing material is carbon black or a Ti-based black pigment.

According to the configuration described above, near infrared light that is emitted from the light emitting region of the light emitting device and could undesirably leak to the light transmissive portion can be adequately absorbed by the light absorbing material.

In the biological body information acquisition apparatus according to the application example described above, it is preferable that the light emitting device has a first electrode and a second electrode so disposed on the first substrate that the electrodes face each other and a light emission function layer disposed between the first electrode and the second electrode, and that the resin layer is provided between the first electrode and the second electrode and so disposed that the resin layer defines a region where the first electrode is in contact with the light emission function layer and encloses an outer edge of the first electrode in a plan view.

The configuration described above does not allow the near infrared light emitted from the light emitting region of the light emitting device to be reflected off the interface between the resin layer and the first electrode or the interface between the resin layer and the second electrode and therefore does not allow the near infrared light to leak toward the light transmissive portion. That is, near infrared light that is emitted from the light emitting region of the light emitting device and could undesirably leak to the light transmissive portion can be reliably absorbed by the resin layer.

In the biological body information acquisition apparatus according to the application example described above, it is preferable that the light emitting device has a reflection layer disposed between the first substrate and the first electrode, and that the first electrode has light transmissivity and is layered on the reflection layer.

According to the configuration described above, near infrared light as illumination light emitted from the light emitting device can be efficiently extracted through the second electrode. That is, the light emitting section can efficiently illuminate a biological body.

In the biological body information acquisition apparatus according to the application example described above, the light emitting device may have a reflection layer disposed between the first substrate and the first electrode, and the first electrode may have light transmissivity and may be layered on the reflection layer via an interlayer insulating film.

According to the configuration described above, adjustment of the film thickness of the interlayer insulating film between the reflection layer and the first electrode allows extraction of near infrared light as the illumination light with enhanced optical intensity at a specific wavelength in the near infrared wavelength region through the second electrode.

It is preferable that the biological body information acquisition apparatus according to the application example described above further includes a light collecting section superimposed on the light emitting section, and the light collecting section preferably includes a light transmissive second substrate and a collector lens provided on the second substrate on a surface thereof facing the light emitting section and in a position where the collector lens faces the light transmissive portion.

According to the configuration described above, when a biological body is brought onto the side where the light collecting section is present relative to the light emitting section, light reflected off an illuminated biological body can be collected with the collector lens and guided to the light receiving device. That is, a biological body information acquisition apparatus capable of acquiring bright, clear biological body information can be provided.

It is preferable that the biological body information acquisition apparatus according to the application example described above further includes a light blocking section disposed between the light emitting section and the imaging section, and the light blocking section preferably includes a light transmissive third substrate, a light blocking layer provided on the third substrate on a surface thereof facing the imaging section, and an opening formed in the light blocking layer and in a position where the opening faces the light receiving device.

According to the configuration described above, the light blocking layer of the light blocking section can block stray light produced in the course of passage of light reflected off an illuminated biological body from the light emitting section to the imaging section and when the light passes through the interface between members having different refractive indices and is reflected off or refracted by the interface. That is, influence of the stray light on the light reflected off the illuminated biological body can be further reduced, whereby a biological body information acquisition apparatus capable of acquiring clearer biological body information can be provided.

The biological body information acquisition apparatus according to the application example described above may further include a light collecting section and a light blocking section disposed between the light emitting section and the imaging section. The light collecting section may include a light transmissive second substrate and a collector lens provided on the second substrate on a surface thereof opposite the light emitting section and in a position where the collector lens faces the light transmissive portion, and the light blocking section may include a light transmissive third substrate, a light blocking layer provided on the third substrate on a surface thereof facing the imaging section, and an opening formed in the light blocking layer and in a position where the opening faces the light receiving device.

According to the configuration described above, providing the light collecting section and the light blocking section between the light emitting section and the imaging section allows a biological body information acquisition apparatus capable of acquiring bright, clearer biological body information to be provided.

In the biological body information acquisition apparatus according to the application example described above, it is preferable that a center of a light receiving surface of the light receiving device, a center of the opening in the light blocking layer, and a center of the light transmissive portion in the light emitting section roughly coincide with one another in a plan view.

According to the configuration described above, light reflected off an illuminated biological body can be efficiently guided to the light receiving device in the imaging section.

Application Example

An electronic apparatus according to this application example includes the image acquisition apparatus according to the application example described above.

According to this application example, since the image acquisition apparatus capable of acquiring a clear image is provided, an electronic apparatus that uses the acquired image to, for example, identify a user as the subject for security management can be provided.

Application Example

An electronic apparatus according to this application example includes the biological body information acquisition apparatus according to the application example described above.

According to this application example, when a biological body is illuminated with the near infrared light emitted from the light emitting section, for example, the fact that a component in the blood flowing through a blood vessel in the biological body has a property of absorbing near infrared light allows acquisition of the pattern of the blood vessel and biological information, for example, on the concentration of the component contained in the blood. An electronic apparatus capable of identification of a biological body, health management of a human body, and other biological-body-related operation based on biological body information acquired by provision of the biological body information acquisition apparatus can be provided. Instead, as the electronic apparatus, a medial apparatus that identifies a component in the blood and the amount of the component for treatment can be provided.

BRIEF DESCRIPTION OF THE DRAWINGS

The invention will be described with reference to the accompanying drawings, wherein like numbers reference like elements.

Fig. 1 is a perspective view showing the configuration of a portable information terminal as an electronic apparatus.
Fig. 2 is a block diagram showing an electric configuration of the portable information terminal as an electronic apparatus.

Fig. 3 is a schematic perspective view showing the configuration of a sensor section.

Fig.4 is a schematic cross-sectional view showing the structure of the sensor section.

Fig. 5 is a diagrammatic cross-sectional view showing the configuration of each light emitting device.

Fig. 6A is a schematic plan view showing the arrangement of the light emitting devices, light transmissive portions, and light receiving devices, and Fig. 6B is a schematic plan view showing the arrangement of a resin layer and the light transmissive portions.

Fig. 7A is a schematic cross-sectional view showing the structure of a light emitting section, and Fig. 7B is a schematic cross-sectional view for describing the optical relationship between the light emitting section and a light collecting section.

Fig. 8 is a schematic cross-sectional view showing the structures of a light blocking section and an imaging section in the sensor section.

Fig. 9 is a schematic cross-sectional view showing the structure of a sensor section as a biological body information acquisition apparatus according to a second embodiment.

Fig. 10 is a schematic plan view showing the arrangement of light emitting devices and light receiving devices in an image acquisition apparatus according to a third embodiment.

Fig. 11 is a schematic cross-sectional view showing the structure of a light emitting device in a variation.

DESCRIPTION OF EXEMPLARY EMBODIMENTS

Embodiments that embody the invention will be described below with reference to the drawings. The drawings to be used in the description are appropriately enlarged or reduced so that a portion being described is recognizable.

First Embodiment

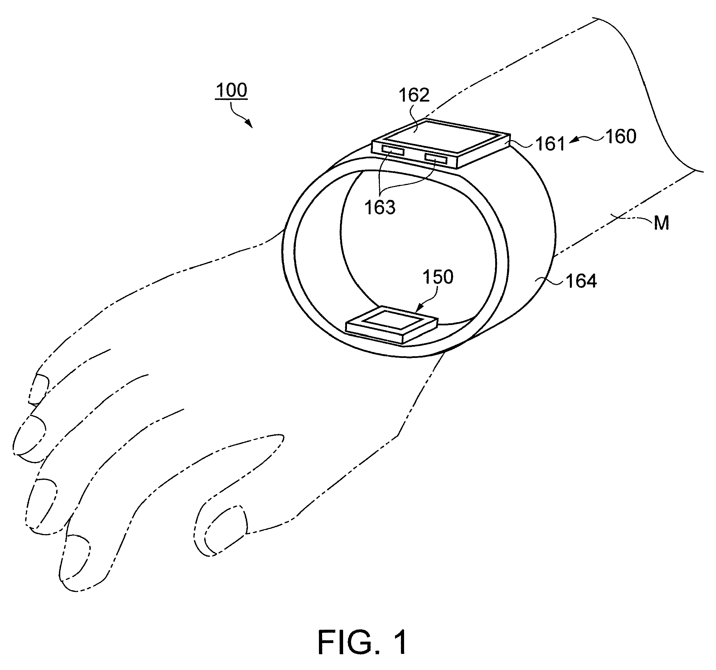

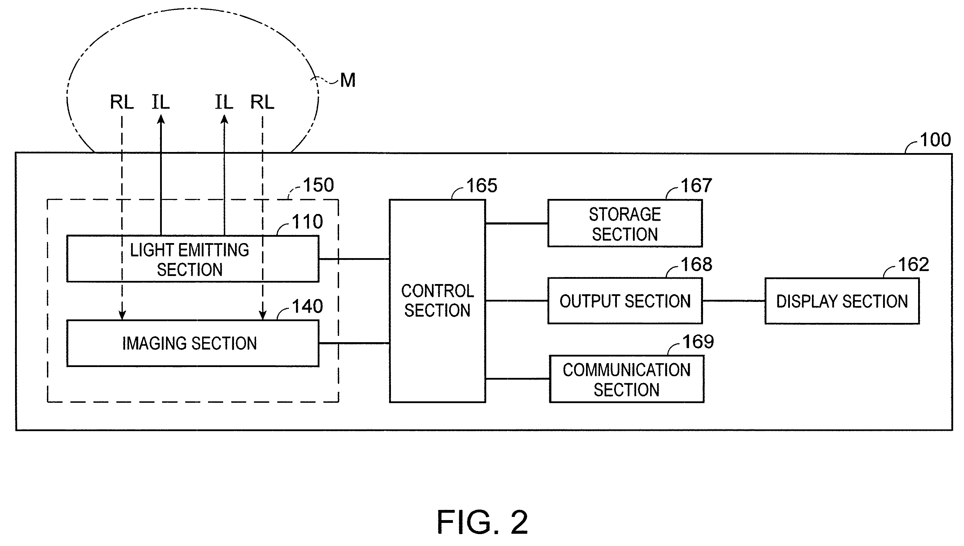

First, as an electronic apparatus according to the present embodiment, a portable information terminal will be described by way of example with reference to Figs. 1 and 2. Fig. 1 is a perspective view showing the configuration of the portable information terminal as the electronic apparatus, and Fig. 2 is a block diagram showing an electric configuration of the portable information terminal as the electronic apparatus.

A portable information terminal 100 as the electronic apparatus according to the present embodiment is an apparatus that is worn around a wrist of a human body M as shown in Fig. 1 and capable of acquiring an image of a blood vessel inside the wrist, a specific component in the blood in the blood vessel, and other types of information. The portable information terminal 100 includes a loop-shaped belt 164, which can be worn around a wrist, a main body section 160, which is attached onto the outer side of the belt 164, and a sensor section 150, which is attached onto the inner side of the belt 164 and in a position where the sensor section 150 faces the main body section 160. The main body section 160 has a main body case 161 and a display section 162, which is incorporated in the main body case 161. In the main body case 161 are incorporated not only the display section 162 but also operation buttons 163, a circuit system (see Fig. 2), such as a control section 165, which will be described later, a battery as a power supply, and other components.

The sensor section 150 is an example of a biological body information acquisition apparatus according to an embodiment of the invention and is electrically connected to the main body section 160 via wiring (not shown in Fig. 1) incorporated in the belt 164. The belt 164 preferably has elasticity in consideration of satisfactory fitness for the human body M.

The thus configured portable information terminal 100 is, when used, so worn that the sensor section 150 is in contact with the palm-side wrist, which is opposite the back of the hand. The thus worn portable information terminal 100 prevents the detection sensitivity of the sensor section 150 from varying depending on the color of the skin.

The portable information terminal 100 according to the present embodiment has a configuration in which the main body section 160 and the sensor section 150 are separately incorporated in the belt 164 and may instead have a configuration in which the main body section 160 and the sensor section 150 are integrated with each other and the integrated section is incorporated in the belt 164.

The portable information terminal 100 includes the control section 165, the sensor section 150, which is electrically connected to the control section 165, a storage section 167, an output section 168, and a communication section 169, as shown in Fig. 2. The portable information terminal 100 further includes the display section 162, which is electrically connected to the output section 168.

The sensor section 150 includes a light emitting section 110 and an imaging section 140. The light emitting section 110 and the imaging section 140 are electrically connected to the control section 165. The light emitting section 110 has light emitting devices each of which emits near infrared light IL having a wavelength that falls within a range from 700 to 2000 nm. The control section 165 drives the light emitting section 110 to cause it to emit the near infrared light IL. The near infrared light IL propagates through the interior of the human body M and is scattered therein. The imaging section 140 can receive part of the near infrared light IL having been scatted in the human body M in the form of reflected light RL.

The control section 165 causes the storage section 167 to store information on the reflected light RL received by the imaging section 140. The control section 165 further causes the output section 168 to process the information on the reflected light RL. The output section 168 converts the information on the reflected light RL into image information on a blood vessel followed by output of the image information and converts the information on the reflected light RL into information on the content of a specific component in the blood followed by output of the content information. The control section 165 still further causes the display section 162 to display the converted image information on the blood vessel and information on the specific component in the blood. The control section 165 further causes the communication section 169 to transmit the information described above to another information processing apparatus. The control section 165 can further receive information, such as a program, from another information processing apparatus via the communication section 169 and cause the storage section 167 to store the information. The communication section 169 may be a wired communication device connected to another information processing apparatus via a wire or a wireless communication device, such as a Blue-tooth-based device (Blue tooth is a registered trademark). The control section 165 may cause the display section 162 to display the program and other types of information stored in the storage section 167 in advance and the current time and other types of information as well as acquired blood-vessel-related and blood-related information. The storage section 167 may be a detachable memory.

Biological body information acquisition apparatus

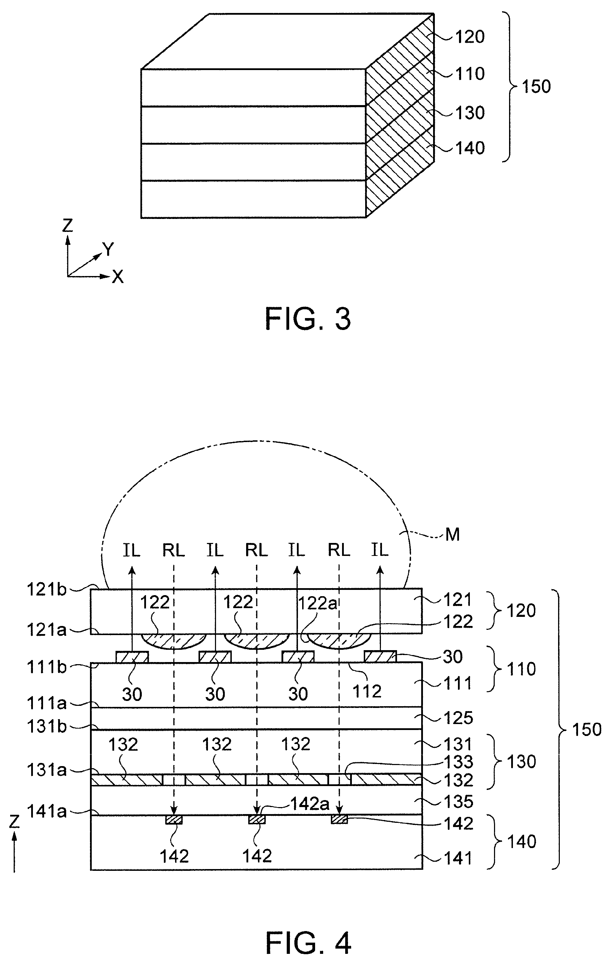

The sensor section 150 as the biological body information acquisition apparatus according to the present embodiment will next be described with reference to Figs. 3 and 4. Fig. 3 is a schematic perspective view showing the configuration of the sensor section, and Fig.4 is a schematic cross-sectional view showing the structure of the sensor section.

The sensor section 150 in the present embodiment includes the light emitting section 110, a light collecting section 120, a light blocking section 130, and the imaging section 140, as shown in Fig. 3. Each of the sections has a plate-like shape, and the light blocking section 130, the light emitting section 110, and the light collecting section 120 are stacked on the imaging section 140 in this order. The sensor section 150 has a case (not shown) that can accommodate the stacked body, which is the stacked sections, and can be attached to the belt 164 of the portable information terminal 100. In the following description, directions are defined as follows: The direction along one side of the stacked body is called an X direction; the direction along another side perpendicular to the one side is called a Y direction; and the thickness direction of the stacked body is called a Z direction. Further, a view from the side where the light collecting section 120 is present and along the Z direction is called a plan view.

The sensor section 150 includes the imaging section 140, and the light blocking section 130, the light emitting section 110, and the light collecting section 120 sequentially stacked on the imaging section 140, as shown in Fig. 4. The light emitting section 110 has a device substrate 111, as a first substrate, which has a plurality of light emitting devices 30 provided on a surface 111b facing the light collecting section 120. A substrate main body of the device substrate 111 is made, for example, of a light transmissive glass or plastic material. The plurality of light emitting devices 30 are arranged on the device substrate 111 at predetermined intervals, and portions between adjacent light emitting devices 30 form light transmissive portions 112. In the following description, a light transmissive substrate refers to a substrate made, for example, of a glass or plastic material, and the term "light transmissive" means transmittance of 85% or higher at least at a representative wavelength of light emitted from the light emitting devices 30.

The light collecting section 120, which is superimposed on the light emitting section 110, has a light transmissive substrate 121 as a second substrate and a plurality of collector lenses 122 provided on one surface 121a of the substrate 121. The light collecting section 120 and the light emitting section 110 are so bonded to each other that a convex lens surface 122a of each of the collector lenses 122 faces the light emitting section 110. The light collecting section 120 and the light emitting section 110 are further so bonded to each other that the optical center of each of the collector lenses 122 is located on the optical axis of the reflected light RL that passes through the corresponding light transmissive portion 112 of the light emitting section 110. In other words, the intervals at which the light transmissive portions 112 are arranged in the light emitting section 110 are basically equal to the intervals at which the collector lenses 122 are arranged in the light collecting section 120. Bonding the light collecting section 120 to the light emitting section 110 provides a structure that protects the light emitting devices 30 and prevents moisture and oxygen and other gases that could undesirably lower the light emission function from externally entering the light emitting devices 30. That is, the light collecting section 120 has not only a function of collecting the reflected light RL incident on the collector lenses 122 but also a function of protecting and encapsulating the light emitting devices 30. Instead, an encapsulating layer that encapsulates the light emitting devices 30 may be provided on the device substrate 111.

The light emitting devices 30 are so configured on the device substrate 111 that they emit the near infrared light IL toward the light collecting section 120 and can therefore illuminate the human body M brought on another surface 121b of the substrate 121 of the light collecting section 120. The light transmissive portions 112 on the device substrate 111 are provided to guide the reflected light RL, which has been reflected off the interior of the illuminated human body M and incident on the light emitting section 110, to the imaging section 140, which is a layer below the light emitting section 110. The light transmissive portions 112 are disposed between the light emitting devices 30 arranged adjacent to each other. The positional relationship between the light emitting devices 30 and the light transmissive portions 112 in the plan view will be described later in detail.

The light blocking section 130 is disposed between the imaging section 140 and the light emitting section 110. The light blocking section 130 has a light transmissive substrate 131 as a third substrate and a light blocking layer 132 provided on a surface 131a, which is a surface of the substrate 131 and faces the imaging section 140. The light blocking layer 132 has openings 133 formed therein in the positions corresponding to the light transmissive portions 112 arranged in the light emitting section 110. The light blocking section 130 is so disposed between the light emitting section 110 and the imaging section 140 that only the reflected light RL having passed through the openings 133 is guided to light receiving devices 142 but the remaining reflected light RL is blocked by the light blocking layer 132. The light blocking layer 132 is formed of a metal film made, for example, of a light-blocking metal, such as Cr, or an alloy thereof, or a resin film containing a light absorbing material capable of absorbing at least near infrared light.

The light blocking section 130 and the light emitting section 110 are so disposed that they face each other via a light transmissive layer 125. Specifically, the light transmissive layer 125 is a space formed of a vacuum layer or an air layer. In other words, a surface 111a, which is a surface of the light emitting section 110 and on which no light emitting devices 30 are provided, and a surface 131b, which is a surface of the light blocking section 130 and on which no light blocking layer 132 is provided, are so disposed that the surfaces 111a and 131b face each other with a predetermined distance therebetween, and the light blocking section 130 and the light emitting section 110 are bonded to each other in a vacuum or atmospheric environment.

The imaging section 140 is an image sensor for near infrared light and has a substrate 141 and the plurality of light receiving devices 142 provided on a surface 141a, which is a surface of the substrate 141 and faces the light blocking section 130. Each of the light receiving devices 142 can, for example, be a CCD, a CMOS device, or any other photosensor. The substrate 141 can, for example, be a glass epoxy substrate or a ceramic substrate on which the light receiving devices 142 can be mounted or a semiconductor substrate in which the light receiving devices 142 can be directly formed, and the substrate 141 has an electric circuit (not shown) to which the light receiving devices 142 are connected. The plurality of light receiving devices 142 are disposed on the surface 141a of the substrate 141 and in the positions corresponding to the openings 133 arranged in the light blocking section 130.

It is known that the sensitivity of a photosensor used as each of the light receiving devices 142 varies with the wavelength of light. For example, the sensitivity of a CMOS sensor is higher for visible light than for the near infrared light IL. When a CMOS sensor receives not only the near infrared light IL (reflected light RL) but also visible light, the CMOS sensor outputs the visible light in the form of noise. Filters that remove light, for example, in a visible wavelength range (400 to 700 nm) may be disposed in correspondence with light transmissive portions 112 in the light emitting section 110 or the openings 133 in the light blocking section 130.

The imaging section 140 and the light blocking section 130 are so disposed that they face each other with a predetermined distance therebetween and bonded to each other via a light transmissive adhesive layer 135. In the present embodiment, the materials of the substrate 131 of the light blocking section 130 and the adhesive layer 135 are so selected that the refractive index n2 of the substrate 131 and the refractive index n3 of the adhesive layer 135 are roughly equal to each other. For example, the substrate 131 of the light blocking section 130 is a quartz glass substrate (refractive index n2 is about 1.53), and the adhesive layer 135 is an epoxy-based resin (refractive index n3 is about 1.55).

The sensor section 150 is not necessarily configured as described above. For example, since the reflected light RL having passed through the light transmissive portions 112 could undesirably be reflected off and attenuated by the interface between members through which the reflected light RL passes, the light blocking section 130 and the light emitting section 110 may be so bonded to each other that the surface 131b, which is a surface of the substrate 131 of the light blocking section 130 and faces the light emitting section 110, is in contact with the surface 111a, which is a surface of the device substrate 111 and faces the light blocking section 130. This configuration further achieves a more reliable positional relationship between the openings 133 and the light transmissive portions 112 in the thickness direction (Z direction).

Light emitting devices

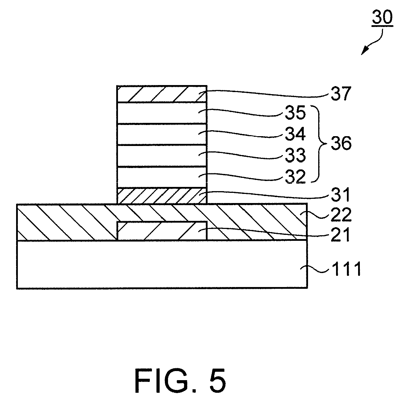

The light emitting devices 30 will next be described with reference to Fig. 5. Fig. 5 is a diagrammatic cross-sectional view showing the configuration of each of the light emitting devices.

Each of the light emitting devices 30 has a reflection layer 21 provided on the device substrate 111 and having light reflectivity, an anode 31 having light transmissivity and serving as a first electrode, a cathode 37 having light transmissivity and serving as a second electrode, and a light emission function layer 36, which is provided between the anode 31 and the cathode 37, as shown in Fig. 5. An interlayer insulating film 22, which adjusts the distance between the reflection layer 21 and the anode 31, is provided between the reflection layer 21 and the anode 31. The light emission function layer 36 has a hole injecting/transporting layer 32, a light emitting layer 33, an electron transporting layer 34, and an electron injecting layer 35 sequentially stacked from the side where the anode 31 is present. Each of the light emitting devices 30 emits light as follows: Holes injected from the anode 31 and electrons injected from the cathode 37 are recombined with each other in the light emitting layer 33; and energy radiated at the time of the recombination is emitted in the form of light. The light emitting layer 33 contains a light emitting material made of an organic semiconductor material, and each of the light emitting devices 30 is therefore called an organic EL (EL: electroluminescence) device. Light emitted from the light emitting layer 33 passes through the cathode 37 and exits out thereof. Part of the emitted light passes through the anode 31, is reflected off the reflection layer 21, passes through the anode 31 again, and exits through the cathode 37. That is, most of the light emitted from the light emitting layer 33 can be extracted through the cathode 37. The thus configured light emitting device 30 is called a top-emission device.

Reflection layer

The reflection layer 21 can be made of a metal having light reflectivity, for example, Al (aluminum), Ag (silver), or an alloy thereof. In consideration of light reflectivity and productivity, preferable examples of the alloy include a combination of Al (aluminum) and Cu (copper) and a combination of Al (aluminum) and Nd (neodymium). The film thickness of the reflection layer 21 is set, for example, at 200 nm in consideration of the light reflectivity.

Anode

The anode 31 is formed, for example, of an ITO film or any other transparent conductive film having a large work function in consideration of readiness of hole injection. The film thickness of the anode 31 is set, for example, at 15 nm in consideration of the light transmissivity.

Cathode

The cathode 37 is made, for example, of an alloy of Ag and Mg and formed by controlling the film thickness in such a way that both light reflectivity and light transmissivity are provided. The film thickness of the cathode 37 is, for example, 20 nm. The cathode 37 is not necessarily formed of an alloy layer made of Ag and Mg and may, for example, have a multilayer structure in which a layer made of Mg is layered on an alloy layer made of Ag and Mg. The configuration formed of the reflection layer 21, the anode 31, and the cathode 37 described above allows part of the light emitted from the light emission function layer 36 of each of the light emitting devices 30 is repeatedly reflected off both the cathode 37 and the reflection layer 21 and outputted with enhanced intensity of light having a specific wavelength determined based on the optical distance between the cathode 37 and the reflection layer 21. That is, each of the light emitting devices 30 has an optical resonance structure that enhances the intensity of light having a specific wavelength. The interlayer insulating film 22 provided between the reflection layer 21 and the anode 31 is provided to adjust the optical distance in the optical resonance structure and is made, for example, of silicon oxide.

Light emitting layer

The light emitting layer 33 of the light emission function layer 36 contains a light emitting material (organic semiconductor material) that allows light emission in the near infrared range (700 to 2,000 nm). Examples of the light emitting material may include a thiadiazole-based compound, a selenadiazole-based compound, or any other known light emitting material. In addition to the light emitting material, a host material to which the light emitting material is added (host material which carries light emitting material) as a guest material (dopant) is used. The host material has a function of causing holes and electrons to be recombined with each other to produce exciters and transferring the energy of the exciters to the light emitting material (Forster transfer or Dexter transfer) to excite the light emitting material. The light emission efficiency can thus be increased. The host material is used, for example, after the light emitting material as a guest material is doped as a dopant to the host material.

A particularly preferable host material is a quinolinolato metal complex or an acene-based organic compound. Among acene-based materials, an anthracene-based material and a tetracene-based material are preferable, and a tetracene-based material is more preferable. When the host material of the light emitting layer 33 contains an acene-based material, electrons can be efficiently transferred from an electron transporting material in the electron transporting layer 34, which will be described later, to the acene-based material in the light emitting layer 33.

Further, an acene-based material excels in resistance against electrons and holes. An acene-based material also excels in thermal stability. An acene-based material can therefore prolong the life of the light emitting devices 30.

Further, when the light emitting layer 33 is formed by using vapor deposition, an acene-based material, which excels in thermal stability, prevents decomposition of the host material due to heat generated at the time of film deposition. The light emitting layer 33 can therefore be formed with excellent film quality. As a result, the light emission efficiency of the light emitting devices 30 can be increased and the life thereof can be prolonged also in this regard.

Further, an acene-based material, which does not tend to emit light by itself, prevents the host material from affecting the spectrum of the light emitted from the light emitting devices 30.

The content of the light emitting material (the amount of doped light emitting material) in the light emitting layer 33, which contains the light emitting material and the host material, preferably ranges from 0.01 to 10 wt%, more preferably 0.1 to 5 wt%. The content of the light emitting material within any of the ranges described above allows optimization of the light emission efficiency.

The average thickness of the light emitting layer 33 is not limited to a specific value and preferably ranges from about 1 to 60 nm, more preferably about 3 to 50 nm.

Hole injecting/transporting layer

The hole injecting/transporting layer 32 contains a hole injecting/transporting material for improving readiness of hole injection into the light emitting layer 33 and readiness of hole transport through the light emitting layer 33. Examples of the hole injecting/transporting material may include an aromatic amine compound having a skeleton part of which is selected from a phenylenediamine-based compound, a benzidine-based compound, and a terphenylene diamine-based compound.

The average thickness of the thus formed hole injecting/transporting layer 32 is not limited to a specific value and preferably ranges from about 5 to 200 nm, more preferably about 10 to 100 nm.

In each of the light emitting devices 30, the layer provided between the anode 31 and the light emitting layer 33 is not necessarily only the hole injecting/transporting layer 32. For example, the hole injecting/transporting layer 32 may instead be formed of a plurality of layers including a hole injecting layer into which holes are readily injected from the anode 31 and a hole transporting layer through which holes are readily transported to the light emitting layer 33. Further, a layer having a function of blocking electrons that leak from the light emitting layer 33 toward the anode 31 may further provided between the anode 31 and the light emitting layer 33.

Electron transporting layer

The electron transporting layer 34 has a function of transporting electrons injected from the cathode 37 via the electron injecting layer 35 to the light emitting layer 33. Examples of the material of which the electron transporting layer 34 is made (electron transporting material) may include a phenanthroline derivative, such as 2,9-dimethyl-4,7-diphenyl-1,10-phenanthroline (BCP), a quinoline derivative, such as an organic metal complex having a ligand made of tris(8-hydroxyquinolinato)aluminum (Alq3) or any other 8-quinolinol or a derivative thereof, an azaindolizine derivative, an oxadiazole derivative, a perylene derivative, a pyridine derivative, a pyrimidine derivative, a quinoxaline derivative, a diphenylquinone derivative, and a nitro-substituted fluorene derivative, and the electron transporting layer 34 can be made of a combination of one or more of the derivatives.

The electron transporting layer 34, when it is made of a combination of two or more of the electron transporting materials described above, may be made of a mixture material that is a mixture of two or more electron transporting materials or may be formed of a laminate of a plurality of layers made of different electron transporting materials.

In particular, when the host material of the light emitting layer 33 is made of a tetracene derivative, the electron transporting layer 34 preferably contains an azaindolizine derivative, more preferably an azaindolizine derivative having an anthracene skeleton in each molecule. In this case, electrons can be efficiently transferred from the anthracene skeleton in each azaindolizine derivative molecule to the host material.

The average thickness of the electron transporting layer 34 is not limited to a specific value and preferably ranges from about 1 to 200 nm, more preferably about 10 to 100 nm.

The layer provided between the light emitting layer 33 and the electron injecting layer 35 is not necessarily only the electron transporting layer 34. For example, the electron transporting layer 34 may instead be a plurality of layers including a layer into which electrons are readily injected from the electron injecting layer 35 and a layer through which electrons are readily transported to the light emitting layer 33 or a layer that controls the amount of electrons to be injected into the light emitting layer 33. Further, a layer having a function of blocking holes that leak from the light emitting layer 33 toward the electron injecting layer 35 may further be provided between the light emitting layer 33 and the electron injecting layer 35.

Electron injecting layer

The electron injecting layer 35 has a function of improving the efficiency at which electrons are injected from the cathode 37.

Examples of the material of which the electron injecting layer 35 is made (electron injecting material) may include a variety of inorganic insulating materials and a variety of inorganic semiconductor materials.

Examples of the inorganic insulating materials may include an alkali metal chalcogenide (oxide, sulfide, selenide, and telluride), an alkali earth metal chalcogenide, a halide of an alkali metal, and a halide of an alkali earth metal, and the electron injecting layer 35 can be made of a combination of one or more of the inorganic insulating materials described above. Forming the electron injecting layer (EIL) by primarily using the materials described above allows improvement in electron injection. In particular, an alkali metal compound (such as alkali metal chalcogenide and halide of alkali metal) has a very small work function, and forming the electron injecting layer 35 by using an alkali metal compound allows the resultant light emitting devices 30 to emit high-luminance light.

Examples of the alkali metal chalcogenide may include $Li_2O$, LiO, $Na_2S$, $Na_2Se$, and NaO.

Examples of the alkali earth metal chalcogenide may include CaO, BaO, SrO, BeO, BaS, MgO, and CaSe.

Examples of the halide of an alkali metal may include CsF, LiF, NaF, KF, LiCl, KCl, and NaCl.

Examples of the halide of an alkali earth metal may include $CaF_2$, $BaF_2$, $SrF_2$, $MgF_2$, and $BeF_2$.

Examples of the inorganic semiconductor materials may include an oxide, a nitride, or an oxynitride containing at least one of elements Li, Na, Ba, Ca, Sr, Yb, Al, Ga, In, Cd, Mg, Si, Ta, Sb, and Zn, and the electron injecting layer 35 can be made of a combination of one or more of the inorganic semiconductor materials described above.

The average thickness of the electron injecting layer 35 is not limited to a specific value and preferably ranges from about 0.1 to 1000 nm, more preferably about 0.2 to 100 nm, still more preferably about 0.2 to 50 nm.

The electron injecting layer 35 may be omitted depending on the materials, thicknesses, and other parameters of the cathode 37 and the electron transporting layer 34.

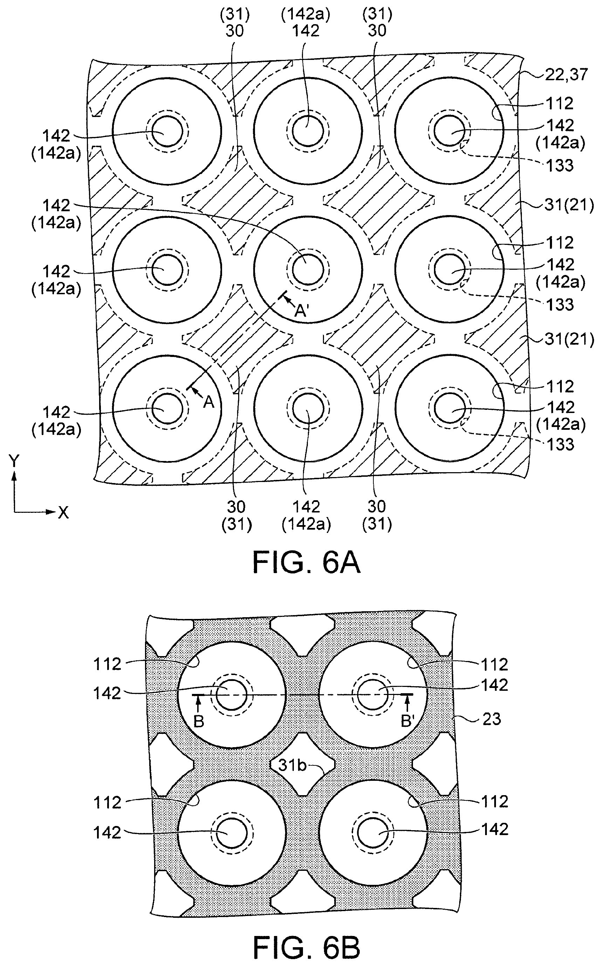

The arrangement of the light emitting devices 30, the light transmissive portions 112, and the light receiving devices 142 in the sensor section 150 will next be described with reference to Figs. 6A and 6B. Fig. 6A is a schematic plan view showing the arrangement of the light emitting devices, the light transmissive portions, and the light receiving devices, and Fig. 6B is a schematic plan view showing the arrangement of a resin layer and the light transmissive portions.

The light receiving devices 142, to which the reflected light RL from the human body M is guided, are arranged at predetermined intervals in the X and Y directions in the form of a matrix, as shown in Fig. 6A. A light receiving surface 142a of each of the light receiving devices 142 has a circular shape. The light transmissive portions 112, which guide the reflected light RL to the light receiving devices 142, each have a circular shape around the respective light receiving devices 142 so that the reflected light RL is uniformly and evenly guided to the light receiving surfaces 142a. The openings 133 in the light blocking section 130 are arranged within the respective light transmissive portions 112 and around the respective light receiving devices 142 and have a circular shape larger than that of the light receiving surfaces 142a.

Each of the light emitting devices 30 disposed between adjacent light transmissive portions 112 therefore has a roughly diamond plan-view shape surrounded by arcs. The plan-view shape of each of the light emitting devices 30 is defined by the anode 31 and a barrier section 23. Specifically, the plan-view shape of the anode 31 is a roughly diamond shape. The barrier section 23, which corresponds to the resin layer in an embodiment of the invention and contains a light absorbing material, is so provided that the barrier section 23 overlaps with an outer edge 31a of the anode 31, as shown in Fig. 6B, and so disposed that the barrier section 23 surrounds the region where the anode 31 is in contact with the light emission function layer 36. That is, the barrier section 23 defines light emitting regions 31b of the light emitting devices 30. Each of the light emitting regions 31b therefore has a roughly diamond plan-view shape that is one-size smaller than the plan-view shape of the anode 31. Each of the light emitting regions 31b defined by the barrier section 23 does not necessarily have a roughly diamond plan-view shape and may instead have a circular, rectangular, or any other polygonal plan-view shape.

The reflection layer 21 and the anode 31 are provided independently for each of the plurality of light emitting devices 30. On the other hand, the interlayer insulating film 22, which covers the reflection layers 21, is provided over the plurality of reflection layers 21. The cathode 37 is provided as a common electrode that extends over the plurality of light emitting devices 30.

As described above, the sensor section 150 in the present embodiment includes a plurality of light emitting devices 30 and a plurality of light receiving devices 142 with four light emitting devices 30 arranged around one light receiving device 142 (light transmissive portion 112). In other words, four light receiving devices 142 (light transmissive portions 112) are arranged around one light emitting device 30. In the imaging section 140, the number of light receiving devices 142 arranged in the X and Y directions in the form of a matrix is preferably, for example, at least 240x240=57,600 from a viewpoint of accurate acquisition of biological body information.

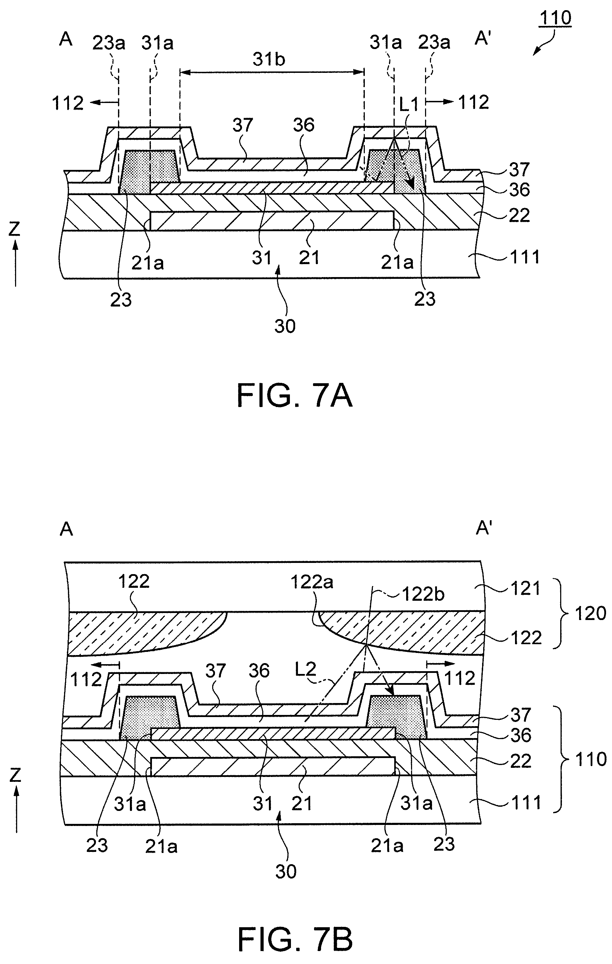

A specific structure of the light emitting section 110 will next be described with reference to Figs. 7A and 7B. Fig. 7A is a schematic cross-sectional view showing the structure of the light emitting section, and Fig. 7B is a schematic cross-sectional view for describing the optical relationship between the light emitting section and the light collecting section. In detail, Figs. 7A and 7B are schematic cross-sectional views showing the structures of one of the light emitting devices 30 and the corresponding light transmissive portion 112 taken along the line A-A', which is shown in Fig. 6A and passes through the anode 31 in a direction inclined by 45 degrees.

The light emitting section 110 has the light emitting devices 30 formed on the device substrate 111 and the light transmissive portions 112, as shown in Fig. 7A. A film made of a light reflective metal, for example, Al (aluminum), or an alloy containing the metal is first formed on the device substrate 111, and the film is then so patterned that the reflection layer 21 is formed. The interlayer insulating film 22, which covers the reflection layer 21, is then formed over the entire surface of the device substrate 111. A transparent conductive film made, for example, of ITO is deposited on the interlayer insulating film 22, and the transparent conductive film is so patterned so that the anode 31 is formed above the reflection layer 21. The patterning is so performed that the outer edge 31a of the anode 31 roughly coincides with an outer edge 21a of the corresponding reflection layer 21 or the outer edge 31a of the anode 31 is located inside the outer edge 21a of the reflection layer 21. The barrier section 23 is so formed that it overlaps with the outer edge 31a of the anode 31. The barrier section 23 as the resin layer can be made of an insulating material, for example, a photosensitive resin material containing a light absorbing material. In the present embodiment, a photosensitive resin film is formed to a film thickness within a range from 1.0 to 2.0 $\mu$m over roughly the entire surface of the device substrate 111, and the photosensitive resin film is so patterned that the barrier section 23 is formed. The patterned barrier section 23 surrounds the light emitting region 31b, where the anode 31 is in contact with the light emission function layer 36. The patterned barrier section 23 has an end portion 23a, which is located on the side opposite the light emitting region 31b and encloses the outer edge 31a of the anode 31 and the outer edge 21a of the reflection layer 21 in the plan view. That is, the outer edge of the light transmissive portion 112 is defined by the end portion 23a of the barrier section 23, resulting in the circular light transmissive portion 112 in the plan view, as described above.

Examples of the light absorbing material contained in the barrier section 23 (photosensitive resin film) may include carbon black and a Ti-based black pigment. Use of the light absorbing material reliably allows the barrier section 23 to absorb the near infrared light IL incident thereon. Only one light absorbing material is not necessarily used, and two or more light absorbing materials may be used. For example, a combination of a material that absorbs light in the visible wavelength region and a material that absorbs light in the near infrared wavelength region may be used.

The light emission function layer 36 is then formed over roughly the entire surface of the device substrate 111 on which the barrier section 23 has been formed. The light emission function layer 36 includes the hole injecting/transporting layer 32, the light emitting layer 33, the electron transporting layer 34, and the electron injecting layer 35, as described above, and each of the layers is formed by using vacuum deposition or any other vapor deposition, followed by sequential stacking of the deposited layers. Each of the layers is not necessarily formed by using vapor deposition, and part of the layers may be formed by using liquid deposition. The cathode 37, which covers the light emission function layer 36, is then formed over roughly the entire surface of the device substrate 111, for example, by using an alloy of Ag and Mg in vacuum deposition or any other vapor deposition in such a way that the light emission function layer 36 has both light reflectivity and light transmissivity. An encapsulating layer that covers the cathode 37 may further be formed. The encapsulating layer is made of an inorganic material or an organic material having low gas permeability.

As described above, each of the light emitting devices 30 includes the reflection layer 21, the interlayer insulating film 22, the anode 31, the light emission function layer 36, and the cathode 37. Each of the light transmissive portions 112, which are formed on the device substrate 111 and between the light emitting devices 30, includes the interlayer insulating film 22, the light emission function layer 36, and the cathode 37. Although not shown in Fig. 7A, a pixel circuit that performs electrical switching control on the anode 31 of the light emitting device 30 to cause current to flow through the region between the anode 31 and the cathode 37 is provided between a substrate main body of the device substrate 111 and the reflection layer 21. The pixel circuit includes a transistor as a switching device, storage capacitance, and wiring that connects the transistor and the capacitance to each other. The reflection layer 21 functions as a relay electrode that allows the pixel circuit to provide the anode 31 with potential.

According to the structure of the light emitting section 110 described above, most of the light emitted from the light emitting region 31b of the top-emission light emitting device 30 exits through the cathode 37. On the other hand, in the region which is outside the light emitting region 31b and where the barrier section 23 is provided, light L1 emitted from the light emission function layer 36 could undesirably be reflected off the surface of the anode 31 and further reflected off the interface between the light emission function layer 36 and the cathode 37 and leak through and out of the outer edge 31a of the anode 31, as indicated by the arrow with a chain double-dashed line in Fig. 7A. However, since the barrier section 23, which is provided between the anode 31 and the cathode 37, contains the light absorbing material, the barrier section 23 absorbs the light L1, which could undesirably be leakage light (stray light).

Further, according to the structure of the light emitting section 110, light L2 emitted from the light emitting region 31b could undesirably be reflected off the lens surface 122a of a collector lens 122 and leak toward the light transmissive portion 112, which is located outside the light emitting region 31b, as indicated by the arrow with a chain double-dashed line in Fig. 7B. The light L2 (stray light) is also absorbed by the barrier section 23. That is, a structure in which stray light (light L1, L2) other than the reflected light RL is unlikely to be incident on the light transmissive portions 112 between the light emitting devices 30 is achieved.

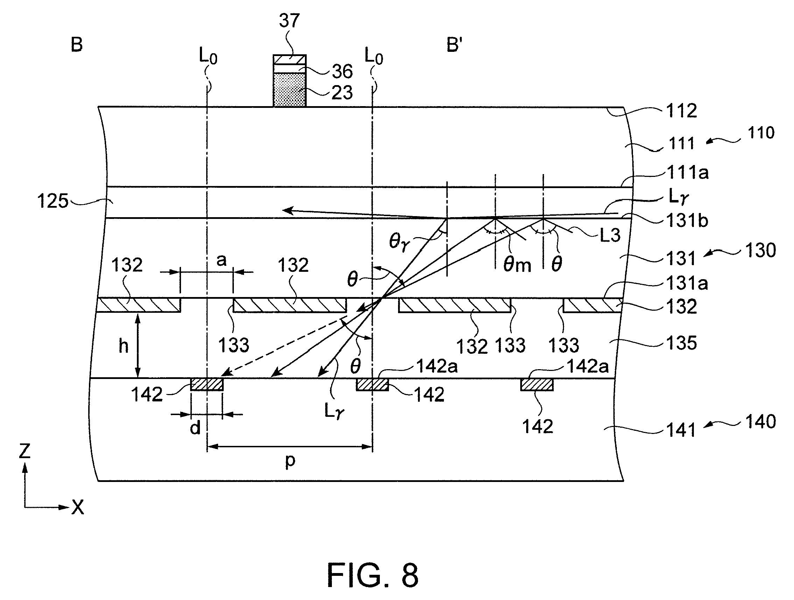

The positional relationship between the light receiving devices 142 in the imaging section 140 and the openings 133 in the light blocking layer 132 in the light blocking section 130 will next be described with reference to Fig. 8. Fig. 8 is a schematic cross-sectional view showing the structures of the light blocking section and the imaging section in the sensor section. In detail, Fig. 8 is a schematic cross-sectional view showing the structures of the light emitting section 110, the light blocking section 130, and the imaging section 140 taken along the line B-B', which is shown in Fig. 6B and crosses light receiving devices 142 adjacent to each other in the X direction.

The light blocking section 130 is layered on the imaging section 140 via the adhesive layer 135, and the light emitting section 110 is further layered on the light blocking section 130 via the light transmissive layer 125, as shown in Fig. 8. The light transmissive layer 125, which is a vacuum layer or an air layer as described above, is also called a space 125 in some cases. The center of the light receiving surface 142a of each of the light receiving devices 142 and the center of the corresponding opening 133 in the light blocking layer 132 are located on an optical axis $L_0$, which passes through the center of the corresponding light transmissive portion 112 , which has a circular plan-view shape. In practice, when the imaging section 140, the light blocking section 130, and the light emitting section 110 are stacked on each other, the center of each of the light transmissive portions 112, the center of the light receiving surface 142a of the corresponding light receiving device 142, and the center of the corresponding opening 133 in the light blocking layer 132 only need to be located within a manufacturing process tolerance range with respect to the optical axis $L_0$ in a plane perpendicular thereto.

The reflected light RL, which originates from the human body M illuminated with the light from the light emitting section 110 and collected by the collector lenses 122, is incident on the light transmissive portions 112, as described above. The reflected light RL passes through the openings 133 in the light blocking section 130 and is incident on the light receiving devices 142 in the imaging section 140. In other words, the light transmissive portions 112, the openings 133, and the light receiving devices 142 are so relatively positioned that the reflected light RL collected by the collector lenses 122 is incident on the light receiving devices 142 in consideration of the focal length of the collector lenses 122.

On the other hand, since the space 125, the refractive index of which is smaller than the refractive index of the substrate 131, is present between the device substrate 111 of the light emitting section 110 and the substrate 131 of the light blocking section 130, light incident from the space 125 on the surface 131b of the substrate 131 is refracted by the substrate 131, so that the entire refracted light is not necessarily incident on the light receiving devices 142.

For example, consider two light receiving devices 142 adjacent to each other in the X direction in the imaging section 140 (one on the left and the other at the center in Fig. 8). Light L3 that enters the opening 133 facing the other light receiving device 142 could undesirably reach the one light receiving device 142, as indicated by the arrow with a solid line in Fig. 8. The light L3 is also recognized as the stray light that affects the reflected light RL incident on the one light receiving device 142. In the present embodiment, the size of the openings 133 relative to the size of the light receiving surfaces 142a of the light receiving devices 142 and the positional relationship between the light receiving devices 142 and the openings 133 are so defined that the light L3 (stray light) is unlikely to be incident on the one light receiving device 142.

Specifically, let d be the diameter of the light receiving surfaces 142a of the light receiving devices 142, a be the diameter of the openings 133, p be the intervals at which the light receiving devices 142 are arranged, n1 be the refractive index of the space (light transmissive layer) 125, n2 be the refractive index of the substrate 131, and h be the distance between the light receiving devices 142 and the light blocking layer 132, and the diameter d, the diameter a, the arrangement intervals p, and the distance h are so defined that the following Numerical Expression (1) is satisfied.

$$\mathrm{Arctan}((p-a/2-d/2)/h) \geq \mathrm{Arcsin}(n1/n2) \quad (1)$$

According to Snell' law, $\theta m = \mathrm{Arcsin}(n1/n2)$ represents a critical angle $\theta m$ in a case where light is incident from the substrate 131, which forms the light blocking section 130 and has the refractive index n2, on the space 125, which has the refractive index n1, as shown in Fig. 8. On the other hand, $\theta = \mathrm{Arctan}((p-a/2-d/2)/h)$ represents an angle $\theta$ in a case where the light L3 incident through one of the openings 133 adjacent to each other in the light blocking section 130 (opening 133 at the center in Fig. 8) is incident on the light receiving surface 142a of the light receiving device 142 facing another opening 133 (opening 133 on the left in Fig. 8). The angle of incident $\theta\gamma$ of light $L\gamma$ incident from the space 125 on the substrate 131, refracted by the substrate 131, and incident on the one opening 133 in the light blocking section 130 is smaller than the critical angle $\theta m$. That is, when the angle of incidence $\theta\gamma$ is slightly smaller than the critical angle $\theta m$, as an optical path of the light $L\gamma$ incident on the opening 133, an optical path from the space 125 into the substrate 131 exists. When the angle of incidence $\theta\gamma$ is equal to the critical angle $\theta m$ and the total reflection condition is satisfied, no optical path from the space 125 into the substrate 131 exists, but assuming that the optical path virtually exists, the virtual optical path is parallel to the surface 131b of the substrate 131. As described above, when the angle $\theta$ described above is equal to or greater than the critical angle $\theta m$, the light L3 incident on the one opening 133 in the light blocking section 130 is not incident on the light receiving surface 142a of the light receiving device 142 facing the other opening 133. In the present embodiment, in which the refractive index n2 of the substrate 131 is roughly equal to the refractive index n3 of the adhesive layer 135, as described above, provided that the angle of incidence of the light L3 incident on the opening 133 is the angle $\theta$, the angle of incidence of the light incident through the opening 133 on the light receiving surface 142a of the light receiving device 142 is also roughly the same angle $\theta$.

In the present embodiment, the parameters described above have, for example, the following values: The diameter d of the light receiving surfaces 142a of the light receiving devices 142 is 10 $\mu$m; the diameter a of the openings 133 is 16 $\mu$m; the distance h between the light receiving devices 142 and the light blocking layer 132 is 100 $\mu$m; the intervals p at which the light receiving devices 142 are arranged in the X direction is 100 $\mu$m; the refractive index n1 of the space 125 is 1.0; and the refractive index n2 of the substrate 131 is about 1.53. Therefore, according to Numerical Expression (1) described above, the total reflection angle $\theta m$ is about 40.8 and the angle $\theta$ is about 41.0, whereby the amount of stray light that affects the reflected light RL incident on the light receiving devices 142 is reduced. In the present embodiment, the space 125 is a vacuum layer or an air layer and the refractive index n1 is therefore 1.0, but the space 125 or the light transmissive layer 125 is not limited to a space. As long as the light transmissive layer 125 is a layer made of a light transmissive material having a smaller refractive index n1 than the refractive index n2 of the substrate 131, the total reflection angle $\theta m$ can be identified.

According to the sensor section 150 in the first embodiment, the stray light resulting from the light emitted from the light emitting section 110 (near infrared light) is less likely to be incident through the openings 133 on the light receiving surfaces 142a of the light receiving devices 142. The reflected light RL incident on the light receiving surfaces 142a is therefore less likely to be affected by the stray light, whereby the sensor section 150 provided in the first embodiment is capable of acquiring clear biological body information.

Further, the portable information terminal 100 as the electronic apparatus including the sensor section 150 is capable of accurately acquiring an image of a blood vessel in the human body M who wears the portable information terminal 100, information on a specific component in the blood in the blood vessel, and other types of information. For example, reducing the influence of the stray light allows accurate acquisition of a change in absorbance due to a change in concentration of a specific component in the blood, leading to accurate quantitative evaluation of the specific component.

The stray light described above includes the light L1 and L2, which is the near infrared light IL that is emitted from the light emitting devices 30 but is not applied to the human body M and could undesirably leak toward the light transmissive portions 112 adjacent to the light emitting regions 31b, as shown in Figs. 7A and 7B. The stray light described above further includes, as shown in Fig. 8, in which the one light receiving device 142 and the other light receiving device 142 adjacent to each other in the X direction are considered, the light L3, which is incident through the opening 133 facing the other light receiving device 142 on the one light receiving device 142. The example shown in Fig. 8 relates to the light receiving devices 142 adjacent to each other in the X direction and the openings 133 adjacent to each other in the X direction, and the same holds true for the light receiving devices 142 adjacent to each other in the Y direction and the openings 133 adjacent to each other in the Y direction.

Second Embodiment

Biological body information acquisition apparatus

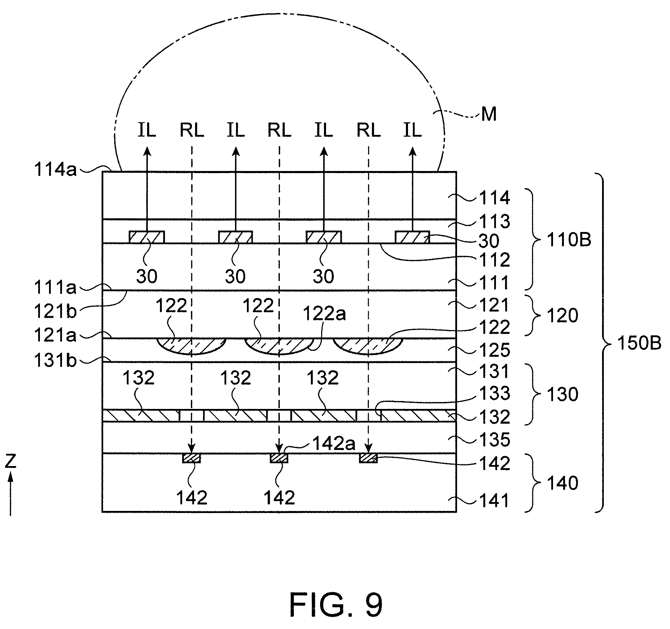

A biological body information acquisition apparatus according to a second embodiment will next be described with reference to Fig. 9. Fig. 9 is a schematic cross-sectional view showing the structure of a sensor section as the biological body information acquisition apparatus according to the second embodiment. A sensor section 150B as the biological body information acquisition apparatus according to the second embodiment differs from the sensor section 150 in the first embodiment described above in terms of the configuration of the light emitting section 110 and the arrangement of the light collecting section 120. Therefore, the same configurations as those of the sensor section 150 in the first embodiment have the same reference characters and will not be described in detail.

The sensor section 150B in the present embodiment includes a light emitting section 110B, the light collecting section 120, the light blocking section 130, and the imaging section 140, as shown in Fig. 9. Each of the sections has a plate-like shape, and the light blocking section 130, the light collecting section 120, and the light emitting section 110B are stacked on the imaging section 140 in this order. The sensor section 150B has a case (not shown) that can accommodate the stacked body, which is the stacked sections, and can be attached to the belt 164 of the portable information terminal 100 as the electronic apparatus described in the first embodiment.

The light emitting section 110B includes the device substrate 111, on which a plurality of light emitting devices 30, which emit the near infrared light IL, an encapsulating layer 113, which encapsulates the light emitting devices 30 so that moisture or other substances do not enter the light emitting devices 30, and a protective substrate 114, which is so disposed that it faces the device substrate 111 via the encapsulating layer 113. The light transmissive portions 112 are provided on the device substrate 111 and between the light emitting devices 30 adjacent to each other at predetermined intervals.

The protective substrate 114 is a light transmissive substrate, for example, formed of a cover glass plate or made of a plastic material. The human body M is brought to come into contact with one surface 114a of the protective substrate 114.

The encapsulating layer 113 is made, for example, of a thermosetting epoxy-based resin or acrylic resin and has light transmissivity.

The light collecting section 120 has a light transmissive substrate 121 and a plurality of collector lenses 122 provided on one surface 121a of the substrate 121. The light collecting section 120 and the light emitting section 110B are so bonded to each other that a convex lens surface 122a of each of the collector lenses 122 faces the light blocking section 130. The light collecting section 120 and the light emitting section 110B are further so bonded to each other that the optical center of each of the collector lenses 122 is located on the optical axis of the reflected light RL that passes through the center of the corresponding light transmissive portion 112 having a circular plan-view shape. Further, a surface 111a, which is a surface of the device substrate 111 and on which no light emitting devices 30 are provided is in contact with a surface 121b, which is a surface of the substrate 121 and on which no collector lenses 122 are provided. In other words, the intervals at which the light transmissive portions 112 are arranged in the light emitting section 110B are basically equal to the intervals at which the light collector lenses 122 are arranged in the light collecting section 120.

A light transmissive layer 125 is provided between the light collecting section 120 and the light blocking section 130. The light transmissive layer 125 is a space having a predetermined thickness in the Z direction, and the space is a vacuum layer or an air layer. The light transmissive layer 125 is therefore called the space 125 also in the present embodiment. In other words, the surface 121a, on which the collector lenses 122 are provided in the light collecting section 120, and a surface 131b in the light blocking section 130 are so disposed that the surfaces face each other with a predetermined distance therebetween, and the light collecting section 120 and the light blocking section 130 are bonded to each other in a vacuum or atmospheric environment.

The light blocking section 130 and the imaging section 140 are so disposed that they face each other with a predetermined distance therebetween and bonded to each other via a light transmissive adhesive 135. Also in the present embodiment, the materials of the substrate 131 of the light blocking section 130 and the adhesive layer 135 are so selected that the refractive index n2 of the substrate 131 and the refractive index n3 of the adhesive layer 135 are roughly equal to each other.

The arrangement of the light emitting devices 30, the collector lenses 122, the openings 133, and the light receiving devices 142 in the plan view in the sensor section 150B in the present embodiment is basically the same as the arrangement described with reference to Figs. 6A and 6B in the first embodiment described above. That is, the light blocking section 130, the light collecting section 120, and the light emitting section 110B are so stacked on the imaging section 140 that the center of each of the collector lenses 122, the center of the corresponding opening 133 in the light blocking layer 132, and the center of the light receiving surface 142a of the corresponding light receiving device 142 are located on the optical axis of the reflected light RL passing through the center of the corresponding light transmissive portion 112 having a circular plan-view shape.

Further, the relationship among the diameter d of the light receiving surfaces 142a of the light receiving devices 142 and the intervals p at which the light receiving devices 142 are arranged in the imaging section 140, the diameter a of the openings 133 in the light blocking section 130, the distance h between the light receiving devices 142 and the light blocking layer 132, the refractive index n1 of the space 125, and the refractive index n2 of the substrate 131 in the light blocking section 130 is the same as the relationship shown in Fig. 8 in the first embodiment described above and satisfies Numerical Expression (1) described above.

According to the sensor section 150B in the second embodiment, the stray light resulting from the light emitted from the light emitting section 110B (near infrared light) is less likely to be incident through the openings 133 on the light receiving surfaces 142a of the light receiving devices 142, as the sensor section 150 in the first embodiment described above.

In particular, disposing the light collecting section 120 between the light blocking section 130 and the light emitting section 110B avoids the situation in which the light emitted from the light emitting devices 30 is reflected off the lens surfaces 122a of the collector lenses 122 and incident on the light transmissive portions 112, resulting in stray light. The reflected light RL incident on the light receiving surfaces 142a of the light receiving devices 142 is therefore less likely to be affected by the stray light, whereby the sensor section 150B provided in the second embodiment is capable of acquiring clear biological body information.

Therefore, the portable information terminal 100 as the electronic apparatus including the sensor section 150B is capable of accurately acquiring an image of a blood vessel in the human body M who wears the portable information terminal 100, information on a specific component in the blood in the blood vessel, and other types of information.

Third Embodiment

Image acquisition apparatus

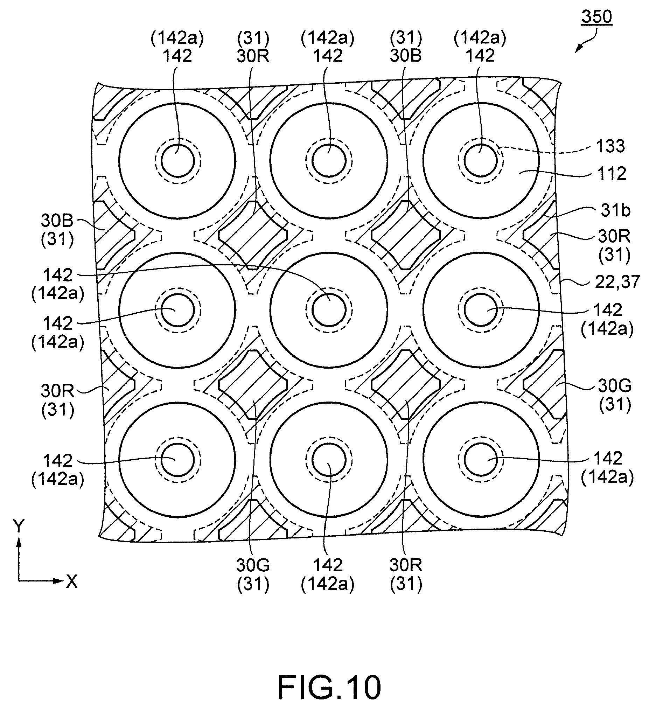

An image acquisition apparatus according to a third embodiment will next be described with reference to Fig. 10. Fig. 10 is a schematic plan view showing the arrangement of light emitting devices and light receiving devices in the image acquisition apparatus according to the third embodiment. An image acquisition apparatus 350 according to the third embodiment includes a light emitting section having a configuration different from that of the light emitting section 110 in the sensor section 150 as the biological body information acquisition apparatus according to the first embodiment described above. The same configurations as those in the sensor section 150 have the same reference characters and will not be described in detail.

The image acquisition apparatus 350 according to the present embodiment includes the light emitting section 110, the light collecting section 120, the light blocking section 130, and the imaging section 140, as the sensor section 150 in the first embodiment described above. Each of the sections has a plate-like shape, and the light blocking section 130, the light emitting section 110, and the light collecting section 120 are stacked on the imaging section 140 in this order. The image acquisition apparatus 350 may instead have a basic configuration that is the same as the basic configuration of the sensor section 150B in the second embodiment. That is, the image acquisition apparatus 350 may instead be a stacked body in which the light blocking section 130, the light collecting section 120, and the light emitting section 110B are stacked on the imaging section 140 in this order. In the present embodiment, in which the light emitting section 110 has a configuration different from that in the first embodiment, the light emitting section is called a light emitting section 110C in the following description.

The image acquisition apparatus 350 has light receiving sections 142 arranged at predetermined intervals in the X and Y directions in the imaging section 140, as shown in Fig 10. The image acquisition apparatus 350 further has, in the light emitting section 110C, light transmissive portions 112, each of which is circular around the center of the corresponding light receiving section 142 in the plan view, and three types of light emitting devices 30R, 30G, and 30B, which arranged between the light transmissive portions 112 disposed at predetermined intervals in the X and Y directions.

The light emitting devices 30R, 30G, and 30B are each an organic EL device and operate as follows: The light emitting device 30R emits red (R) light; the light emitting device 30G emits green (G) light; and the light emitting device 30B emits blue (B) light.

A device row in which the light emitting device 30R and the light emitting device 30G are alternately arranged in the X direction and a device row in which the light emitting device 30B and the light emitting device 30R are alternately arranged in the X direction are alternately arranged in the Y direction. A device column in which the light emitting device 30R and the light emitting device 30B are alternately arranged in the Y direction and a device column in which the light emitting device 30G and the light emitting device 30R are alternately arranged in the Y direction are thus formed. That is, one light emitting device 30B, one light emitting device 30G, and two light emitting device 30R are arranged around one light receiving section 142 (light transmissive portion 112). The arrangement of the three types of light emitting devices 30R, 30G, and 30B is not limited to the arrangement described above. Further, light emitting devices that provide emitted light colors different from red (R), green (G), and blue (B) may instead be arranged.

The configuration of the reflection layer 21, the anode 31, the barrier section 23, the cathode 37, and other components in each of the light emitting devices 30R, 30G, and 30B is basically the same as the configuration of the light emitting devices 30 in the first embodiment described above, and light that is emitted from the light emitting regions 31b and could undesirably leak toward the light transmissive portions 112 is absorbed by the barrier section 23 containing a light absorbing material and is therefore not incident on the light transmissive portions 112. Further, the relationship among the diameter d of the light receiving surfaces 142a of the light receiving devices 142 and the intervals p at which the light receiving devices 142 are arranged in the imaging section, the diameter a of the openings 133 in the light blocking section 130, the distance h between the light receiving devices 142 and the light blocking layer 132, the refractive index n1 of the space 125, and the refractive index n2 of the substrate 131 in the light blocking section 130 satisfies Numerical Expression (1) in the first embodiment described above.

The film thickness of the interlayer insulating film 22 disposed between the reflection layer 21 and the anode 31 is preferably set for each of the light emitting devices 30R, 30G, and 30B, which emit light of different specific wavelengths, from a viewpoint of increasing the intensity of light of the specific wavelengths in the optical resonance structure.

According to the image acquisition apparatus 350 of the third embodiment, the stray light resulting from the light emitted from the light emitting section 110C is less likely to be incident through the openings 133 on the light receiving surfaces 142a of the light receiving devices 142. The reflected light incident from a subject illuminated with the light from the light emitting section 110C on the light receiving surfaces 142a of the light receiving sections 142 is therefore less likely to be affected by the stray light, whereby the image acquisition apparatus 350 provided in the third embodiment is capable of acquiring a clear image. Further, since the light emitting section 110C includes the three types of light emitting devices 30R, 30G, and 30B, a color image of the subject can be acquired. Moreover, since each of the light emitting devices 30R, 30G, and 30B can be independently controlled in terms of light emission, an image according to the state of a subject can be acquired.

Using the thus configured image acquisition apparatus 350 in place, for example, of the sensor section 150 in the portable information terminal 100 according to the first embodiment described above and capturing an image of a finger as a subject allows acquisition of fingerprint information. Use of the acquired fingerprint information allows security management in which a person who is operating the apparatus is identified. Further, reduction in the influence of the stray light allows, for example, accurate acquisition of changes in absorbance (at three wavelengths) due to a change in concentration of a specific component in the blood, leading to highly accurate quantitative evaluation of the specific component.

The invention is not limited to the embodiments described above and can be changed as appropriate to the extent that the change does not depart from the substance or spirit of the invention read from the appended claims and the entire specification, and a thus changed image acquisition apparatus and biological body information acquisition apparatus, and an electronic apparatus using the image acquisition apparatus or the biological body information acquisition apparatus also fall within the technical range of the invention. In addition to the embodiments described above, a variety of variations are conceivable. Variations will be described below.

Variation 1

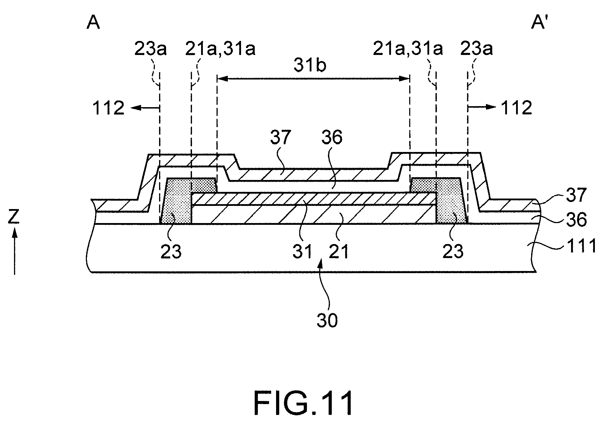

In each of the light emitting devices 30 in the first embodiment described above, the interlayer insulating film 22 is not necessarily disposed between the reflection layer 21 and the anode 31. Fig. 11 is a schematic cross-sectional view showing the structure of a light emitting device in the variation. In detail, Fig. 11 is a schematic cross-sectional view of the light emitting device taken along the line A-A' in Fig. 6A, as in the case of Fig. 7A in the first embodiment described above.

The light emitting device 30 in the variation has an anode 31 having light transmissivity and directly layered on the reflection layer 21 having light reflectivity, as shown in Fig. 11. The barrier section 23 is so formed that it covers the outer edges 21a and 31a of the reflection layer 21 and the anode 31 and at least light emitting region 31b in the anode 31 is exposed. The end portion 23a of the barrier section 23 on the side opposite the light emitting region 31b forms the outer edges of adjacent light transmissive portions 112. According to the structure of the thus configured light emitting device 30 in the variation, light that is emitted from the light emitting region 31b and could undesirably leak to the light transmissive portions 112 outside the light emitting region 31b can be absorbed by the barrier section 23, as in the first embodiment. Further, the reflection layer 21 and the anode 31 can be readily electrically connected to each other.

Variation 2

In each of the embodiments described above, the reflection layer 21 is not necessarily provided independently for each of the light emitting devices. For example, the reflection layer 21 may be so formed that it covers the plurality of light emitting devices 30, and circular light transmissive portions 112 may then be formed by removing portions of the reflection layer 21 that overlap with the light receiving devices 142 in the plan view. In this case, the reflection layer 21 and the anode 31 are electrically separated from each other.

Variation 3

The image acquisition apparatus 350 according to the third embodiment described above does not necessarily include the three types of light emitting devices 30R, 30G, and 30B in the light emitting section 110C. For example, one or two types of light emitting devices capable of emitting light in the visible wavelength region may be provided. Further, a light emitting device that emits light in the visible wavelength region and a light emitting device that emits light in the near infrared wavelength region may be provided. In this case, image information on a subject and information on a biological body in the subject can be acquired.

Variation 4

An electronic apparatus using the sensor section 150 or the sensor section 150B as the biological body information acquisition apparatus is not limited to the portable information terminal 100. For example, a personal computer using the sensor section 150 or 150B can perform biological body authentication in which a person who is using the personal computer can be identified based on an image of a blood vessel. Further, information on a specific component in the user's blood can be acquired.

Further, for example, as a medical apparatus, the invention is applicable to an apparatus that measures the blood pressure, blood sugar, pulse, pulse wave, amount of cholesterol, amount of hemoglobin, in-blood water, amount of in-blood oxygen, and other quantities. Further, the invention allows, along with pigment, measurement of the liver function (detoxification rate), checking of the position of a blood vessel, and checking of a tumor site. Moreover, benign/malignant tumor (melanoma) of skin cancer can be evaluated by an increase in the amount of findings of a specimen. Further, indices of skin age and skin health can be evaluated by comprehensive evaluation of part or all of the items described above.

**Claims**

1. An image acquisition apparatus comprising:

   an imaging section including a light receiving device; and
   a light emitting section that is superimposed on the imaging section and illuminates a subject,
   wherein the light emitting section includes a light transmissive first substrate, a light emitting device provided on the first substrate, a resin layer that defines a light emitting region in the light emitting device, and a light transmissive portion that transmits light reflected off the illuminated subject and guides the reflected light to the light receiving device, and
   the resin layer contains an insulating resin material and a light absorbing material.

2. The image acquisition apparatus according to claim 1,
   wherein the light absorbing material is carbon black or a Ti-based black pigment.

3. The image acquisition apparatus according to claim 1 or 2,
   wherein the light emitting device has a first electrode and a second electrode so disposed on the first substrate that the electrodes face each other and a light emission function layer disposed between the first electrode and the second electrode, and
   the resin layer is provided between the first electrode and the second electrode and so disposed that the resin layer defines a region where the first electrode is in contact with the light emission function layer and encloses an outer edge of the first electrode in a plan view.

4. The image acquisition apparatus according to claim 3,
   wherein the light emitting device has a reflection layer disposed between the first substrate and the first electrode, and
   the first electrode has light transmissivity and is layered on the reflection layer.

5. The image acquisition apparatus according to claim 3,
   wherein the light emitting device has a reflection layer disposed between the first substrate and the first electrode, and
   the first electrode has light transmissivity and is layered on the reflection layer via an interlayer insulating film.

6. The image acquisition apparatus according to any one of the preceding claims,
   further comprising a light collecting section superimposed on the light emitting section,
   wherein the light collecting section includes a light transmissive second substrate and a collector lens provided on the second substrate on a surface thereof facing the light emitting section and in a position where the collector lens faces the light transmissive portion.

7. The image acquisition apparatus according to any one of the preceding claims,
   further comprising a light blocking section disposed between the light emitting section and the imaging section,
   wherein the light blocking section includes a light transmissive third substrate, a light blocking layer provided on the third substrate on a surface thereof facing the imaging section, and an opening formed in the light blocking layer and in a position where the opening faces the light receiving device.

8. The image acquisition apparatus according to any one of the preceding claims,
   further comprising a light collecting section and a light blocking section disposed between the light emitting section and the imaging section,
   wherein the light collecting section includes a light transmissive second substrate and a collector lens provided on the second substrate on a surface thereof opposite the light emitting section and in a position where the collector lens faces the light transmissive portion, and

the light blocking section includes a light transmissive third substrate, a light blocking layer provided on the third substrate on a surface thereof facing the imaging section, and an opening formed in the light blocking layer and in a position where the opening faces the light receiving device.

9. The image acquisition apparatus according to claim 7 or 8,
wherein a center of a light receiving surface of the light receiving device, a center of the opening in the light blocking layer, and a center of the light transmissive portion in the light emitting section roughly coincide with one another in a plan view.

10. A biological body information acquisition apparatus comprising the image acquisition apparatus according to any one of the preceding claims, wherein
the light emitting section illuminates a biological body, and
the light transmissive portion transmits light reflected off the illuminated biological body and guides the reflected light to the light receiving device.

11. An electronic apparatus comprising the image acquisition apparatus according to any one of claims 1 to 9.

12. An electronic apparatus comprising the biological body information acquisition apparatus according to claim 10.

FIG. 1

EP 3 035 244 A1

EP 3 035 244 A1

FIG. 2

FIG. 3

FIG. 4

FIG. 5

FIG. 6A

FIG. 6B

EP 3 035 244 A1

FIG. 7A

FIG. 7B

25

FIG. 8

EP 3 035 244 A1

FIG. 9

FIG.10

A                                                                A'

23a   21a,31a          31b          21a,31a   23a

112 ←                                              → 112

37   36

37
36
111

23        31  21          23

30

Z ↑

# FIG.11

Europäisches
Patentamt

European
Patent Office

Office européen
des brevets

# EUROPEAN SEARCH REPORT

Application Number

EP 15 20 0086

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2014/084405 A1 (TSUCHIYA HITOSHI [JP] ET AL) 27 March 2014 (2014-03-27) * figures * * paragraph [0026] - paragraph [0039] * * paragraph [0061] * | 1-12 | INV. G06K9/00 H01L51/52 H01L27/32 |
| Y,D | & JP 2014 067577 A (SEIKO EPSON CORP) 17 April 2014 (2014-04-17) * abstract * | 1-12 | |
| Y | JP 2006 065305 A (SEMICONDUCTOR ENERGY LAB) 9 March 2006 (2006-03-09) * abstract * * paragraphs [0042], [0047] * | 1-12 | |
| Y | US 2008/169757 A1 (CHANG SHIH-CHANG [TW] ET AL) 17 July 2008 (2008-07-17) * paragraphs [0016], [0017], [0019], [0020]; figures 1,2 * | 5 | |
| Y | US 2012/257031 A1 (TSUCHIYA HITOSHI [JP] ET AL) 11 October 2012 (2012-10-11) * figure 2 * | 8 | TECHNICAL FIELDS SEARCHED (IPC) G06K H01L |

The present search report has been drawn up for all claims

| Place of search | Date of completion of the search | Examiner |
|---|---|---|
| The Hague | 6 May 2016 | De Laere, Ann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 20 0086

This annex lists the patent family members relating to the patent documents cited in the above-mentioned European search report.
The members are as contained in the European Patent Office EDP file on
The European Patent Office is in no way liable for these particulars which are merely given for the purpose of information.

06-05-2016

| Patent document cited in search report | | Publication date | Patent family member(s) | | Publication date |
|---|---|---|---|---|---|
| US 2014084405 | A1 | 27-03-2014 | CN | 103685883 A | 26-03-2014 |
| | | | JP | 2014067577 A | 17-04-2014 |
| | | | US | 2014084405 A1 | 27-03-2014 |
| | | | US | 2015325615 A1 | 12-11-2015 |
| JP 2006065305 | A | 09-03-2006 | JP | 5364227 B2 | 11-12-2013 |
| | | | JP | 2006065305 A | 09-03-2006 |
| US 2008169757 | A1 | 17-07-2008 | TW | 200833166 A | 01-08-2008 |
| | | | US | 2008169757 A1 | 17-07-2008 |
| US 2012257031 | A1 | 11-10-2012 | JP | 5828371 B2 | 02-12-2015 |
| | | | JP | 2012221141 A | 12-11-2012 |
| | | | US | 2012257031 A1 | 11-10-2012 |

EPO FORM P0459

For more details about this annex : see Official Journal of the European Patent Office, No. 12/82

**EP 3 035 244 A1**

## REFERENCES CITED IN THE DESCRIPTION

**Patent documents cited in the description**

- JP 2014067577 A